Europäisches Patentamt

European Patent Office

Office européen des brevets

(19)

(11) Numéro de publication : **0 421 368 B1**

(12) **FASCICULE DE BREVET EUROPEEN**

(45) Date de publication du fascicule du brevet :
**12.05.93 Bulletin 93/19**

(51) Int. Cl.$^5$ : **A61H 1/02**, A63B 23/16,
A63B 21/02

(21) Numéro de dépôt : **90118904.3**

(22) Date de dépôt : **03.10.90**

(54) **Dispositif pour la rééducation d'un membre.**

(30) Priorité : **05.10.89 FR 8913127**

(43) Date de publication de la demande :
**10.04.91 Bulletin 91/15**

(45) Mention de la délivrance du brevet :
**12.05.93 Bulletin 93/19**

(84) Etats contractants désignés :
**AT BE CH DE DK GB IT LI NL SE**

(56) Documents cités :
**EP-A- 0 145 166**
**EP-A- 0 251 437**
**US-A- 4 671 258**
**US-A- 4 830 360**

(56) Documents cités :
**C.M. JACKSON et al.: "55-Nitinol-the alloy
with a memory: Its physical metallurgy, properties, and applications", 1972, pages 1-2,
NASA, Washington, DC, US**

(73) Titulaire : **Nivarox-FAR S.A.
Avenue du Collège 10
CH-2400 Le Locle (CH)**

(72) Inventeur : **Guy, Grenouillet
Rue Capitaine Richardot
F-25130 Villers-Le-Lac (FR)**

(74) Mandataire : **Caron, Gérard et al
ICB Ingénieurs Conseils en Brevets SA
Passage Max. Meuron 6
CH-2001 Neuchâtel (CH)**

EP 0 421 368 B1

## Description

L'invention concerne le domaine du matériel médical ou vétérinaire, et plus particulièrement un dispositif pour la rééducation de membres comprenant un élément moteur en alliage à mémoire de forme. Ce dispositif est destiné notamment à rétablir la fonction articulaire et/ou musculaire d'un membre à la suite d'un accident, d'une immobilisation prolongée ou analogue.

Dans le présent texte, le terme "membre" doit être compris comme désignant toutes parties du corps humain ou animal qui comprend un muscle et/ou une articulation.

On sait que pour recouvrer la fonction articulaire ou musculaire d'un membre ayant subi un traumatisme ou une immobilisation prolongée, il est nécessaire de procéder à la rééducation de ce dernier en lui imposant des séries de mouvements déterminés.

Généralement, la rééducation peut être réalisée de deux façons qui peuvent être indépendantes ou complémentaires consistant soit en l'intervention d'un praticien, soit en l'utilisation d'appareils mécaniques spécifiques à cette rééducation.

Toutefois, dans les deux cas, le patient encore convalescent doit effectuer des déplacements fréquents et à heures fixes chez le praticien ou dans une salle spécialisée ou se trouvent lesdits appareils de rééducation.

Il en résulte donc, d'une part, une fatigue du patient et, d'autre part, des contraintes horaires souvent peu compatibles avec une activité professionnelle.

On connaît également du document US-A-4 830 360 un dispositif pour la rééducation d'un membre tel que défini dans le préambule de la revendication 1 du brevet.

Toutefois ce document présente l'inconvénient de nécessiter une participation active du patient.

De plus, les appareils de rééducation mécaniques utilisés se caractérisent généralement par un coût relativement élevé et sont des appareils compliqués, présentant des structures lourdes et peu aisées à mettre en oeuvre par le patient seul.

L'invention a donc pour but de remédier aux inconvénients analysés plus haut en fournissant un dispositif pour la rééducation d'un membre, simple, léger, peu coûteux et facile à mettre en oeuvre par le patient lui-même.

A cet effet, l'invention a pour objet un dispositif pour la rééducation d'un membre tel que défini dans la revendication 1 du brevet.

Selon un mode préféré de réalisation, ces moyens de liaison comprennent une enveloppe recevant au moins une partie du membre à rééduquer et contenant les éléments moteurs. Cette enveloppe peut être formée notamment d'une gaine ou d'une bande de tissu à la façon d'un pansement.

On sait que les alliages à mémoire de forme ont la propriété de pouvoir subir une transformation réversible d'un premier état de phase cristallographique du type austénitique (haute température HT) à un second état de phase cristallographique du type martensitique (basse température BT) et ainsi de pouvoir être éduqués pour prendre dans certaines conditions une configuration que l'on appelle état de mémoire de forme, ou état mémorisé.

En d'autres termes, si l'on éduque un objet composé d'un tel alliage de sorte qu'il mémorise une configuration dans l'état de phase austénitique (HT), par exemple, et que l'on déforme ultérieurement cet objet pendant qu'il est dans un état de phase martensitique (BT), il reste dans sa configuration déformée. Si par la suite cet objet est chauffé pour être amené à une température à laquelle son état de phase est austénitique (HT), il tend progressivement à reprendre sa configuration non déformée, c'est-à-dire la configuration correspondant à son état mémorisé.

Avec de tels alliages, la transition d'un état de phase à un autre s'effectue dans un sens comme dans l'autre dans un intervalle de température. La température à laquelle la phase austénitique commence à apparaître lors du chauffage de l'alliage est appelée TAs et la température à laquelle la formation de la phase est achevée est appelée TAf (TAf > TAs). De façon similaire, lors du refroidissement de l'alliage, les températures de début et de fin de transformation de phase martensitique sont appelées respectivement TMs et TMf (TMf < TMs).

D'une manière générale, il est à noter que TMs et TMf sont respectivement sensiblement inférieures à TAf et TAs, les intervalles de températures [TAs, TAf] et [TMs, TMf] étant dépendants de la composition de l'alliage.

On notera également que parmi ces alliages, les Ti, Ni ; Ti, Ni, X ; Cn, Al, X ; X appartenant à l'ensemble des dopants métalliques sont particulièrement intéressants.

De tels alliages et leur procédé d'éducation sont notamment décrits dans un article de la NASA, SP 5110 publié en 1972 et intitulé "55 Nitinol, the alloy with a memory : its physical metallurgy properties and applications".

Ainsi l'intégration d'un ou de plusieurs éléments moteurs formés d'un alliage à mémoire de forme, par exemple à un pansement destiné à être rigidement liée à un membre à rééduquer permet, en imposant à cet élément un chauffage et un refroidissement alternés, de réaliser un mouvement déterminé du membre en vue de sa rééducation.

De préférence, on utilisera les propriétés conductrices de l'alliage pour réaliser un chauffage par effet Joule de l'élément moteur, les moyens de commande pouvant être ainsi formés d'une simple source de tension aux bornes desquelles est branché l'élément moteur.

D'autres avantages et caractéristiques de l'invention apparaîtront au cours de la description détaillée qui suit de modes de réalisation donnés à titre illustratif en liaison avec les dessins parmi lesquels :

- la figure 1 est une vue en coupe longitudinale représentant de façon schématique un premier mode de réalisation du dispositif selon l'invention, muni de ses moyens de commande et mis en place sur un doigt dans une première position ;
- la figure 2 est une vue similaire à la figure 1, les moyens de commande ayant été omis, tandis que le doigt se trouve dans une seconde position ;
- la figure 3 est une vue en coupe transversale selon la ligne III-III de la figure 2 ;
- la figure 4 est une vue en coupe longitudinale représentant de façon schématique un second mode de réalisation du dispositif selon l'invention qui est de nouveau muni de ses moyens de commande et mis en place sur un doigt dans une première position ;
- la figure 5 est une vue similaire à la figure 4, mais représentant partiellement le dispositif selon l'invention et dans une seconde position ;
- la figure 6 est une vue en coupe transversale selon la ligne VI-VI de la figure 5 ;
- la figure 7 est une vue de dessus représentant de façon schématique et sans circuit de commande, un troisième mode de réalisation du dispositif selon l'invention ; et
- la figure 8 est une vue en coupe transversale selon la ligne VIII-VIII de la figure 7.

La description détaillée de l'invention va être faite dans le cadre d'une application à la rééducation des articulations d'un doigt, mais il va de soi que l'invention n'est pas limitée aux modes de réalisation décrits ou envisagés. En particulier, le dispositif selon l'invention pourra être avantageusement utilisé pour la rééducation d'autres membres, et dans ce cas, il présentera des configurations différentes qui seront adaptées à la forme du membre sur lequel il sera mis en place.

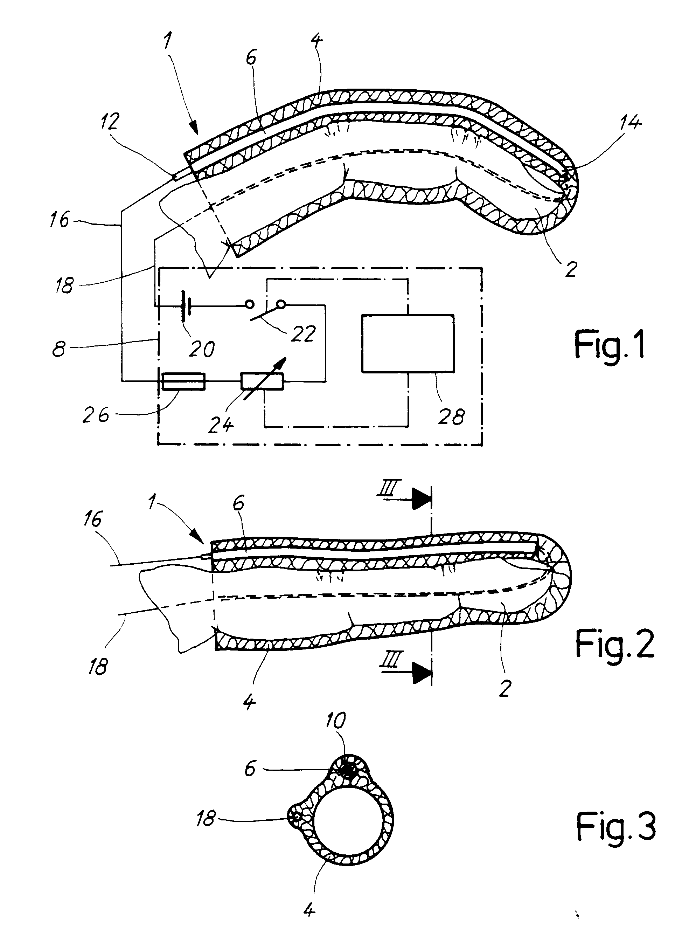

En se référant tout d'abord aux figures 1 à 3, on voit un premier mode de réalisation du dispositif pour la rééducation d'un membre selon l'invention, désigné généralement par la référence numérique 1.

Ce dispositif mis en place sur un doigt 2 comprend une gaine 4 enveloppant sensiblement la totalité du doigt à rééduquer.

Cette gaine 4, réalisée par exemple au moyen d'un élément tissé, comprend un élément moteur 6 en alliage à mémoire de forme relié à un circuit 8 formant les moyens de commande.

L'élément moteur 6 est formé d'un fil en alliage à mémoire de forme éduqué simple effet et dont la forme mémorisée est la forme droite de l'élément moteur 6 représentée à la figure 2.

L'élément moteur 6 qui présente une section droite circulaire, est enrobé dans une matière synthétique 10 (figure 3) électriquement et thermiquement isolante telle qu'un silicone ou analogue.

Comme il ressort clairement des figures 1 à 3, l'élément moteur 6 est intégré au sein d'une paroi de la gaine 4 et s'étend parallèlement à son axe longitudinal sensiblement sur la totalité de la longueur de la gaine.

Bien entendu, selon une variante de réalisation, l'élément moteur peut être rapporté et fixé sur une face externe de l'enveloppe, par exemple à l'aide d'une bande auto-adhésive ou analogue.

Dans le mode réalisation représenté, l'élément moteur se situe sur le dessus du doigt, lorsque le dispositif est mis en place sur doigt, mais il est bien entendu que selon l'éducation de l'élément moteur disposé dans la gaine 4 et la forme de la section de cet élément, le dispositif peut être mis en place de sorte que l'élément moteur se situe en dessous ou sur les côtés du doigt.

Par ailleurs, l'élément moteur 6 a une extrémité proximale 12 et une extrémité distale 14 reliées respectivement par l'intermédiaire de deux liaisons électriques classiques 16, 18 au circuit de commande 8. On notera que les liaisons électriques 16, 18 sont également enrobées d'un matériau électriquement isolant, et de préférence, il s'agit du même matériau que celui enrobant l'élément moteur 6.

Le circuit 8 formant les moyens de commande comprend en série une alimentation en courant continu 20, un interrupteur 22, un dispositif 24 pour faire varier l'intensité du courant circulant dans le circuit et des moyens coupe-circuit 26. Le circuit 8 comprend en outre des moyens de contrôle 28 reliés en parallèle à l'interrupteur 22 et au dispositif 24.

De manière avantageuse, le circuit de commande 8 peut être réalisé à l'aide de composants intégrés disponibles dans le commerce, les moyens de contrôle 28 comprenant notamment un microprocesseur associé à une mémoire programmable. Dans un exemple de réalisation, les moyens de contrôle 28 comprennent des moyens de temporisation afin de programmer et/ou de contrôler automatiquement des cycles de rééducation.

Le fonctionnement du dispositif selon l'invention est le suivant :

Tout d'abord, il est à noter que le dispositif décrit plus haut ne permet, avec un élément moteur éduqué simple effet, qu'une rééducation dite "active". On entend par rééducation "active", une rééducation comportant un effort de la part du patient qui doit tenter d'effectuer un mouvement sans aucune aide extérieure. Dans l'exemple décrit, il s'agit de rééduquer les articulations d'un doigt en demandant au patient de plier son doigt.

Le doigt 2 est enfilé dans la gaine 4, qui se trouve par exemple dans une position correspondant à la position tendue du doigt (figure 2), et dans l'exemple illustré a la forme mémorisée de l'élément moteur 6. L'élément moteur se trouve à ce moment dans un état de phase cristallographique martensitique (basse température ou encore T < TMs). Le patient replie son doigt pour l'amener dans la position représentée à la figure 1 tout en déformant l'élément moteur 6. Une fois le doigt replié et l'élément moteur 6 déformé, le patient ferme le circuit de commande 8 à l'aide de l'interrupteur 22. Cette fermeture provoque la circulation d'un courant à travers l'élément moteur et par conséquent son échauffement par effet Joule, si bien que la température de l'élément moteur s'élève. Dès que cette température dépasse la température TAs, c'està-dire la température à laquelle la phase cristallographique austénitique (haute température) commence à se former, l'élément moteur 6 se déforme progressivement pour reprendre son état mémorisé (figure 2) dès que l'élément moteur atteint une température sensiblement supérieure à TAf.

A ce moment, le circuit de commande peut être ouvert par le patient ou le cas échéant par les moyens de contrôle de sorte que l'élément moteur 6 se refroidit et que son état de phase cristallographique passe de l'état austénitique à l'état martensitique initial. A ce moment, un nouveau cycle peut être effectué.

On comprend donc aisément que dans une première phase le patient fournit un effort pour replier son doigt et que dans une seconde phase l'élément moteur 6 solidaire de la gaine 4 entraîne le doigt dans une position droite.

Une répétition de ce cycle un grand nombre de fois permet au patient d'effectuer sans aide extérieure, simplement et à son rythme, la rééducation de son membre lésé.

Dans l'exemple de réalisation représenté aux figures 1 à 3, on a utilisé un fil en alliage Ti - Ni du type 50-50, le fil présentant un diamètre de D = 2 mm environ et le circuit étant alimenté avec une tension de 3 volts environ . Ceci a permis d'obtenir une force motrice Fm de l'ordre de 3,5 N.

A titre indicatif, avec l'alliage utilisé, on obtient les températures de transition de phase suivantes :

TAs = 60°C ; TAf = 90°C ; TMs = 60°C ; TMf = 40°C ;

Bien entendu, la force qui est fournie par l'élément moteur au membre à rééduquer peut varier selon le membre que l'on désire rééduquer et on notera à ce propos que cette force motrice est essentiellement proportionnelle aux dimensions de l'élément moteur utilisé. Il va donc de soi que pour obtenir une même force motrice, il est possible de remplacer un unique élément moteur par une pluralité d'éléments moteurs de dimensions inférieures et agissant dans le même sens.

Afin que le patient ne se sente pas incommodé en raison d'une température trop élevée lors de l'échauffement de l'élément moteur, on pourra prévoir, entre l'élément moteur et la gaine, une couche supplémentaire réalisée en matériau thermiquement isolant (non représenté).

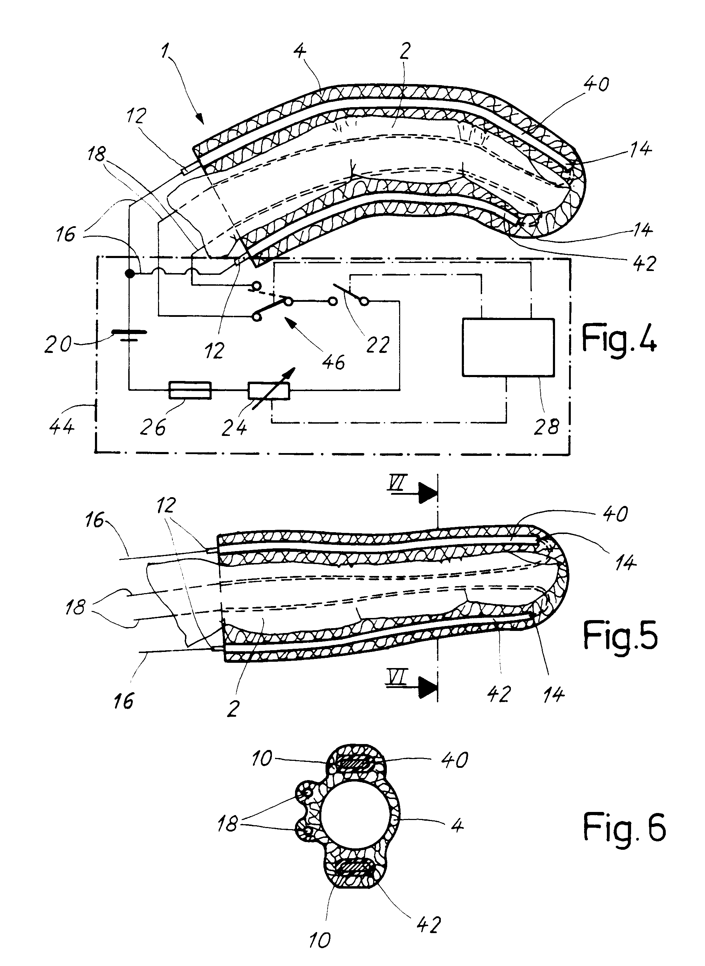

En se référant maintenant aux figures 4 à 6, on voit un second mode de réalisation d'un dispositif selon l'invention dans lequel on a désigné les éléments identiques à ceux décrits précédemment par les mêmes références numériques.

Dans ce second mode de réalisation, le dispositif 1 comprend deux élément moteurs 40, 42 formés d'un alliage à mémoire de forme et réalisés à la façon d'une bande de section droite sensiblement rectangulaire. Ces deux éléments moteurs enrobés dans une matière électriquement et thermiquement isolante sont intégrés dans des parois diamétralement opposées de la gaine 4 et cette dernière est mise en place sur le doigt de sorte qu'ils sont disposés respectivement au dessous et en dessus du doigt. Comme cela a déjà été décrit dans le premier mode de réalisation, chaque élément 40, 42 est relié par l'intermédiaire de liaisons électriques à un circuit de commande 44.

Ce circuit de commande 44 diffère du circuit décrit en liaison avec les figures 1 à 2 en ce qu'il comprend des moyens 46 pour mettre sous tension alternativement l'élément moteur 40 et l'élément moteur 42. Ces moyens 46 sont formés par une bascule bistable reliée, d'une part, à l'alimentation 20 et, d'autre part, à la liaison électrique 18 de chaque élément moteur, la bascule 46 étant par ailleurs reliée aux moyens de contrôle 28.

Les deux éléments moteurs 40, 42 sont dans ce cas éduqués simple effet et présentent des formes mémorisées différentes en ce sens que par exemple, la forme mémorisée (haute température ou phase austénitique) de l'élément moteur 40 est courbe ou "pliée" (figure 4), tandis que la forme mémorisée de l'élément moteur 42 est droite (figure 3).

Ainsi, lorsque l'on alimente alternativement les éléments moteurs 40, 42, ces derniers agissent en opposition si bien qu'un mouvement alternatif du doigt entre sa position pliée (figure 3) et sa position tendue peut être obtenu.

Par cette commande appropriée du dispositif, on peut donc réaliser simplement une rééducation dite "passive", c'est-à-dire sans que le patient fournisse d'effort pour engendrer ces mouvements.

Dans ce cas, les moyens de contrôle 28 sont programmés pour que les éléments moteurs 40, 42 soient systématiquement alimentés successivement et en aucun cas simultanément.

Il va également de soi que ces moyens de contrôle 28 comprennent des moyens de temporisation tels que ceux décrits en liaison avec les figures 1 à 3 pour réaliser des cycles de rééducation adaptés à chaque patient.

Bien entendu, une rééducation passive à l'aide d'un tel dispositif peut être réalisée en remplaçant les deux éléments moteurs 40, 42 éduqués simple effet, par un unique élément moteur en alliage à mémoire de forme éduqué double effet.

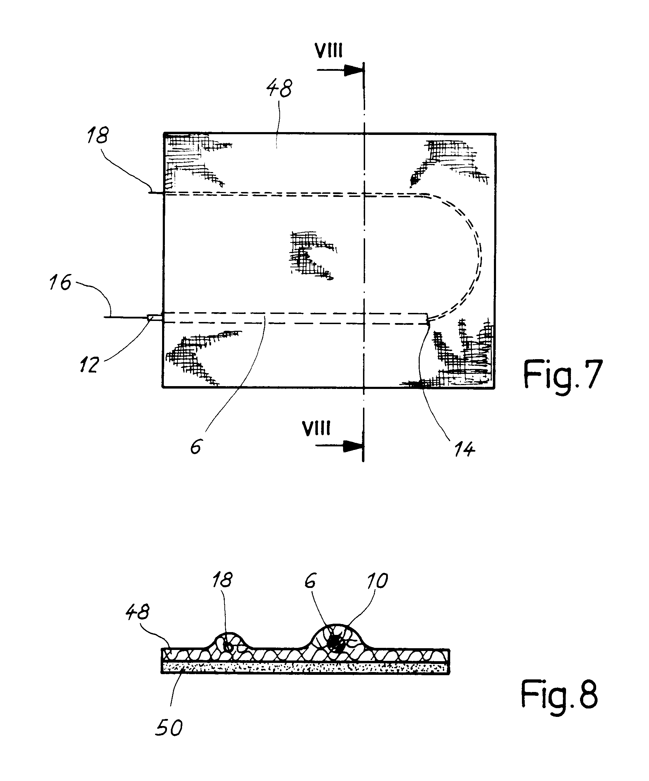

Enfin, en se référant aux figures 7 et 8, on voit un troisième mode de réalisation du dispositif selon l'invention dans lequel un élément moteur unique 6 est intégré dans une bande de tissu 48 munie sur l'une de ses faces d'une matière adhésive 50 à la façon d'un pansement. L'élément moteur 6 est bien entendu relié à un circuit de commande (non représenté) du type de celui décrit en liaison avec les figures 1 à 3.

Selon une variante de réalisation, la matière adhésive 50 peut être remplacée par d'autres moyens de solidarisation au membre à rééduquer, tel que notamment des sangles à boucles, des sangles munies de fermeture Velcro, ou analogues.

On remarquera de manière avantageuse qu'un tel mode de réalisation permet de fixer un même dispositif sur des membres de formes, tailles, sections différentes.

Il est à noter que dans tous les modes de réalisation décrits plus haut, le circuit de commande comprend de préférence un fusible formant les moyens coupe-circuit 26 dans le cas où un court-circuit se produirait dans le dispositif.

Enfin, on notera qu'un tel dispositif présente l'avantage d'être utilisable de façon autonome, c'est-à-dire sans aucune liaison avec un référentiel fixe tel q'un bâti ou analogues et cela, quelle que soit la position dans l'espace du membre à rééduquer.

## Revendications

1. Dispositif pour la rééducation d'un membre (8) comprenant au moins un élément moteur (6;40;42) présentant au moins un état de mémoire de forme correspondant à une configuration déterminée et des moyens de liaison mécanique pour assujetir au moins une partie de l'élément moteur (6;40;42) à une partie du membre (2) à rééduquer, caractérisé en ce que ledit élément est réalisé en alliage à mémoire de forme et en ce que le dispositif comprend des moyens de commande (8) de l'élément moteur pour l'amener dans ledit état de mémoire de forme en réponse à un signal de commande.

2. Dispositif selon la revendication 1, caractérisé en ce que les moyens de liaison comprennent une enveloppe (4; 48) recevant au moins une partie de membre à rééduquer et en ce que chaque élément moteur (6) est intégré à l'enveloppe.

3. Dispositif selon la revendication 1, caractérisé en ce que les moyens de liaison comprennent une enveloppe ayant la configuration d'une gaine (4) pour recevoir au moins une partie de membre à rééduquer et en ce que chaque élément moteur (6) est fixé sur une face externe de l'enveloppe.

4. Dispositif selon l'une quelconque des revendications précédentes, caractérisé en ce que chaque élément moteur est réalisé par un profilé au moins partiellement enrobé d'une matière synthétique (10) électriquement et thermiquement isolante.

5. Dispositif selon l'une quelconque des revendications précédentes, caractérisé en ce que les moyens de commande (8) comprennent une source d'énergie électrique continue (20) branchée aux extrémités (12, 14) de chaque élément moteur (6).

6. Dispositif selon la revendication 5, caractérisé en ce que les moyens de commande (8) comprennent en outre un interrupteur (22) branché entre la source d'énergie électrique continue (20) et une extrémité de chaque élément moteur (6).

7. Dispositif selon la revendication 5 ou 6, caractérisé en ce que les moyens de commande (8) comprennent en outre des moyens (24) pour faire varier l'intensité du courant circulant dans chaque élément moteur.

8. Dispositif selon l'une quelconque des revendications précédentes, caractérisé en ce qu'il comprend aussi des moyens de temporisation (28) contrôlant automatiquement le temps s'écoulant entre deux mises en circuit successives de chaque élément moteur (6).

9. Dispositif selon l'une quelconque des revendications précédentes, caractérisé en ce qu'il comprend en outre des moyens coupe-circuit (26) en cas de surchauffe de chaque élément moteur.

10. Dispositif selon l'une quelconque des revendications précédentes, caractérisé en ce que chaque élément moteur est réalisé en alliage à mémoire de forme éduqué à simple effet.

11. Dispositif selon l'une quelconque des revendications précédentes, caractérisé en ce qu'il comprend deux éléments moteurs (40, 42) disposés de part et d'autre du membre à rééduquer et agissant en sens opposés.

12. Dispositif selon la revendication 11, caractérisé en ce qu'il comprend des moyens (28, 46) pour n'alimenter qu'un élément moteur (40, 42) à la fois.

## Patentansprüche

1. Vorrichtung für die Rehabilitation eines Gliedes (8) umfassend mindestens ein Motorelement (6;40;42), das mindestens einen Formgedächtniszustand aufweist entsprechend einer bestimmten Konfiguration sowie mechanische Verbindungsmittel zum Anbringen mindestens eines Teils des Motorelements (6;40;42) an einer Partie des zu rehabilitierenden Gliedes (2), dadurch gekennzeichnet, daß das Element aus einer Legierung mit Formgedächtnis hergestellt ist und daß die Vorrichtung Steuermittel (8) aufweist für das Motorelement, um es in den genannten Formgedächtniszustand im Ansprechen auf ein Steuersignal zu bringen.

2. Vorrichtung nach Anspruch 1, dadurch gekennzeichnet, daß die Verbindungsmittel eine Hülle (4;48) umfassen, die mindestens eine Partie des zu rehabilitierenden Gliedes aufnimmt und daß jedes Motorelement (6) in die Hülle integriert ist.

3. Vorrichtung nach Anspruch 1, dadurch gekennzeichnet, daß die Verbindungsmittel eine Hülle umfassen mit der Konfiguration eines Überzugs (4) zum Aufnehmen mindestens einer Partie des zu rehabilitierenden Gliedes und daß jedes Motorelement (6) auf einer Außenseite der Hülle befestigt ist.

4. Vorrichtung nach einem der vorangehenden Ansprüche, dadurch gekennzeichnet, daß jedes Motorelement als ein Profil realisiert ist, das mindestens teilweise von einem synthetischen Material (10), das elektrisch und thermisch isolierend ist, umhüllt ist.

5. Vorrichtung nach einem der vorangehenden Ansprüche, dadurch gekennzeichnet, daß die Steuermittel (8) eine elektrische kontinuierliche Energiequelle (20) umfassen, die an die Enden (12,14) jedes Motorelements (6) angeschlossen ist.

6. Vorrichtung nach Anspruch 5, dadurch gekennzeichnet, daß die Steuermittel (8) ferner einen Unterbrecher (22) umfassen, der zwischen die elektrische Dauerenergiequelle (20) und ein Ende jedes Motorelements (6) gelegt ist.

7. Vorrichtung nach Anspruch 5 oder 6, dadurch gekennzeichnet, daß die Steuermittel (8) ferner Mittel (24) umfassen zum sich Ändernlassen der Intensität des Stromes, der in jedem Motorelement zirkuliert.

8. Vorrichtung nach einem der vorangehenden Ansprüche, dadurch gekennzeichnet, daß sie ferner Zeitgebermittel (28) umfaßt, die automatisch die Zeit steuern, die zwischen zwei aufeinanderfolgenden Betriebsphasen jedes Motorelements (6) verstreicht.

9. Vorrichtung nach einem der vorangehenden Ansprüche, dadurch gekennzeichnet, daß sie ferner Schaltkreisunterbrechermittel (26) umfaßt für den Fall der Überhitzung jedes Motorelements.

10. Vorrichtung nach einem der vorangehenden Ansprüche, dadurch gekennzeichnet, daß jedes Motorelement aus einer Legierung mit Formgedächtnis mit Einrichtungswirkung realisiert ist.

11. Vorrichtung nach einem der vorangehenden Ansprüche, dadurch gekennzeichnet, daß sie zwei Motorelemente (40, 42) umfaßt, die beidseits des zu rehabilitierenden Gliedes angeordnet sind und in entgegengesetzter Richtung wirken.

12. Vorrichtung nach Anspruch 11, dadurch gekennzeichnet, daß sie Mittel (28, 46) umfaßt zum Speisen nur jeweils eines Motorelements (40, 42).

## Claims

1. Device for the re-education of a limb (2) having at least one motor element (6; 40; 42) comprising at least one shape memory state corresponding to a given configuration and mechanical attachment means for securing at least a part of the motor element (6; 40; 42) to a part of the limb (2) to be re-educated, characterized in that said element is made of shape memory alloy and in that the device comprises control means (8) for setting the motor element into said shape memory state in response to a control signal.

2. Device according to claim 1, characterized in that the attachment means comprises an envelope (4; 48) accommodating at least a part of a limb to be re-educated and in that each motor element (6) is incorporated in the envelope.

3. Device according to claim 1, characterized in that the attachment means comprises an envelope having the configuration of a sheath (4) for accommodating at least a part of a limb to be re-educated and in that each motor element (6) is attached to an external face of the envelope.

4. Device according to any one of the preceding claims, characterized in that each motor element is formed of a profiled element at least partially coated with an electrically and thermally insulating synthetic material (10).

5. Device according to any one of the preceding claims, characterized in that the control means (8) comprises a direct current source of electrical energy connected to the extremities (12, 14) of each motor element (6).

6. Device according to claim 5, characterized in that the control means (8) furthermore comprises a switch (22) connected between the direct current electrical energy source (20) and one extremity of each motor element (6).

7. Device according to claim 5 or 6, characterized in that the control means (8) furthermore comprises means (24) for varying the strength of the current passing through each motor element.

8. Device according to any one of the preceding claims, characterized in that it furthermore comprises timing means (28) automatically monitoring the expired time between two successive operations of switching-on each motor element (6).

9. Device according to any one of the preceding claims, characterized in that it furthermore comprises circuit-breaking means (26) for the event of overheating of each motor element.

10. Device according to any one of the preceding claims, characterized in that each motor element is made of one way effect educated shape momery alloy.

11. Device according to any one of the preceding claims, characterized in that it comprises two motor elements (40, 42) located on either side of the limb to be re-educated and acting in opposing directions.

12. Device according to claim 11, characterized in that it comprises (28, 46) for energising only one motor element (40, 42) at a time.

Fig.1

Fig.2

Fig.3

Fig. 4

Fig. 5

Fig. 6

Fig.7

Fig.8